# EUROPÄISCHE PATENTSCHRIFT

(11) **EP 3 236 416 B1**
(45) Veröffentlichungstag und Bekanntmachung des Hinweises auf die Patenterteilung: **14.11.2018**
(21) Anmeldenummer: 17162858.9
(22) Anmeldetag: 24.03.2017
(51) Int. Cl.: G06T 5/00, A61B 6/00, G06T 5/10, G06T 5/50, H04N 5/365, H04N 5/32, H04N 5/217, H04N 17/00

(54) **VERFAHREN ZUR KORREKTUR EINES RÖNTGENBILDS AUF EFFEKTE EINES STREUSTRAHLENRASTERS, RÖNTGENEINRICHTUNG, COMPUTERPROGRAMM UND ELEKTRONISCH LESBARER DATENTRÄGER**
METHOD FOR ADJUSTING AN X-RAY IMAGE FOR EFFECTS OF AN ANTI-SCATTER GRID, X-RAY DEVICE, COMPUTER PROGRAM AND ELECTRONICALLY READABLE DATA CARRIER
PROCÉDÉ DE CORRECTION D'UNE RADIOGRAPHIE SUR LES EFFETS D'UNE GRILLE ANTIDIFFUSANTE, ÉQUIPEMENT À RAYONS X, PROGRAMME INFORMATIQUE ET SUPPORT DE DONNÉES LISIBLE ÉLECTRONIQUEMENT

(30) Priorität: 19.04.2016 DE 102016206559
(43) Veröffentlichungstag der Anmeldung: 25.10.2017
(73) Patentinhaber: Siemens Healthcare GmbH, 91052 Erlangen (DE)
(72) Erfinder: Ertel, Dirk, 91301 Forchheim (DE); Bernhardt, Philipp, 91301 Forchheim (DE)

(56) Entgegenhaltungen:
- EP-A2- 0 962 888
- EP-A2- 1 372 108
- EP-A2- 1 505 540
- JP-A- H11 146 277

## Beschreibung

Die Erfindung betrifft ein Verfahren zur Korrektur eines mit einer Röntgeneinrichtung mit einem Streustrahlenraster aufgenommenen Röntgenbilds auf Effekte des Streustrahlenrasters, wobei das Streustrahlenraster eine sich räumlich periodisch wiederholende geometrische Ausgestaltung aufweist und ein ohne Bildgebungsobjekt aufgenommenes Kalibrierungsbild verwendet wird. Daneben betrifft die Erfindung eine Röntgeneinrichtung, ein Computerprogramm und einen elektronisch lesbaren Datenträger.

In der Röntgenbildgebung sind Streustrahlenraster im Stand der Technik bereits weitgehend bekannt, um störende Streustrahlenbeiträge im erhaltenen Röntgenbild zu reduzieren. Insbesondere im Bereich der Angiographie- und Radiographie-Bildgebung werden Streustrahlenraster häufig eingesetzt. Bekannte technische Realisierungen nutzen meist Streustrahlenraster, die äußert dünn herstellbare Bleilamellen zur Röntgenabsorption nutzen. Obwohl die Lamellen eine sehr geringe Stegbreite aufweisen, müssen die durch sie gebildeten Strukturen des Streustrahlenrasters bei der Kalibrierung der bildgebenden Röntgeneinrichtung entsprechend berücksichtigt werden.

Durch die einfache geometrische Struktur und die dünne Stegbreite bei bekannten Streustrahlenrastern kann eine adäquate Bildqualität der letztendlichen Röntgenbilder bereits durch eine konventionelle Kalibrierung sichergestellt werden, in der ein Kalibrierungsbild nur des Streustrahlenrasters, also ohne Bildgebungsobjekt, aufgenommen wird, welches später von dem eigentlichen Röntgenbild subtrahiert werden kann, um die Beiträge des Streustrahlenrasters zu entfernen und ein korrigiertes Röntgenbild zu erhalten. Dann sind in klinischen Röntgenbildern die Lamellen-Strukturen des Streustrahlenrasters nicht mehr erkennbar.

Durch die eingeschränkte geometrische Struktur ist der Anteil der Streustrahlung in den unter Nutzung konventioneller Streustrahlenraster aufgenommenen Röntgenbildern weiterhin recht hoch. Die Bildqualität kann deutlich beeinträchtigt sein, was sich vor allem bei stark absorbierenden Bildgebungsobjekten, beispielsweise stark übergewichtigen Patienten, zeigt.

Daher wurden aktuelle neuartige Ansätze vorgeschlagen, bei denen deutlich komplexere geometrische Strukturen innerhalb von Streustrahlenrastern eingesetzt werden, um das Absorptionsverhalten zu verbessern. Ferner werden dabei auch neuartige Absorptionsmaterialien eingesetzt, welche sich gegebenenfalls nur mit höherer Stegbreite realisieren lassen. Die mithin vorgeschlagenen komplexen Geometrien und gegebenenfalls größeren Stegbreiten erschweren aber eine Kalibrierung beziehungsweise die Verwendung von Kalibrierungsbildern deutlich.

Zu diesem Effekt kommt hinzu, dass bei vielen Röntgeneinrichtungen, beispielsweise bei C-Bogen-Röntgeneinrichtungen, die den Röntgenstrahler und den Röntgendetektor umfassende Aufnahmeanordnung in verschiedene Stellungen gebracht werden kann, um das Bildgebungsobjekt, insbesondere einen Patienten, aus unterschiedlichen Projektionsrichtungen aufnehmen zu können. Dabei können mechanische Effekte, beispielsweise durch anderweitig wirkende Schwerkraft, zum Tragen kommen, wenn unterschiedliche Aufnahmegeometrien im Vergleich zu dem Kalibrierungsbild verwendet werden. Während dies bei Streustrahlenrastern mit einfacheren Strukturen und/oder äußerst dünnen Bleilamellen ein einen eher geringen Einfluss hatte, kann bei komplexeren geometrischen Strukturen und/oder größeren Stegbreiten eine stärkere Einschränkung der Bildqualität auftreten. Derartige Änderungen der Aufnahmegeometrien können auch durch Alterungseffekte der Röntgeneinrichtung bedingt sein.

EP 1 505 540 A1 betrifft ein Verfahren zur Entfernung von Gitterlinien in einem radiographischen Bild. Dabei wird ein anhand einer gemessenen Grundfrequenz und wenigstens einer harmonischen Frequenz parametrierter Filter verwendet, um aus einem mit dem Filter gefilterten Bild ein Gittermodell zu erzeugen und zur Korrektur zu verwenden.

EP 1 372 108 A2 betrifft ein Verfahren zur Detektion und Abschwächung von Artefakten aufgrund eines Streustrahlengitters. Eine zweidimensionale dynamische Korrelation auf dem Bild wird genutzt, um Gitterartefakte zu detektieren. In Abhängigkeit der Gitterfrequenz und des Abschwächungsniveaus wird ein Filter zur Korrektur des Bildes parametriert.

EP 0 962 888 A2 offenbart ein Verfahren zur Entfernung von Gitterlinienartefakten in einem Röntgenbild. Dabei wird das Fourier-Spektrum zur Detektion der Gitterlinienfrequenz analysiert und Spektraldomänenfilterung zur Entfernung der Gitterlinien eingesetzt.

JP H11 146277 A offenbart ein diagnostisches Röntgensystem, bei dem vorgeschlagen wird, ein Nichtgleichförmigkeitsdatenbild ohne Objekt bzw. mit einem Phantom aufzunehmen und dieses von Bildern mit einem Objekt abzuziehen, um Gitterartefakte zu vermeiden.

Der Erfindung liegt daher die Aufgabe zugrunde, ein Verfahren zur verbesserten Verringerung von von einem Streustrahlenraster herrührenden Bildartefakten in Röntgenbildern anzugeben.

Zur Lösung dieser Aufgabe ist bei einem Verfahren der eingangs genannten Art erfindungsgemäß vorgesehen, dass das Kalibrierungsbild und das Röntgenbild mittels einer Transformation in den Ortsfrequenzraum transformiert werden, im Ortsfrequenzraum ein Übereinstimmungsmaß zwischen dem Röntgenbild und dem Kalibrierungsbild optimierende Veränderungen des Kalibrierungsbildes beschreibende Anpassungsparameter ermittelt werden, zur Korrektur das angepasste Kalibrierungsbild von dem Röntgenbild subtrahiert wird und das aus dieser Subtraktion erhaltene, korrigierte Röntgenbild durch Anwendung des Inversen der Transformation wieder in den Ortsraum zurücktransformiert wird.

Dieses Verfahren ermöglicht mithin eine adaptive Bildkorrektur zur Verringerung von durch Streustrahlenraster hervorgerufenen Artefakten, wobei die Korrektur mit besonderem Vorteil im Frequenzraum durchgeführt wird. Durch eine entsprechende Transformation werden spezifische Eigenschaften des Streustrahlenrasters ausgenutzt, so dass eine adäquate Korrektur der insbesondere medizinischen Bilddaten des Röntgenbildes erfolgen kann. Dies ermöglicht eine Anwendung von Streustrahlenrastern mit komplexer Struktur und neuartigen Materialien auch im Feld der interventionellen Radiologie sowie sonstigen medizinischen Anwendungen, bei denen eine besonders hohe Bildqualität erforderlich ist. Die hierdurch ermöglichte Verwendung verbesserter Streustrahlenraster reduziert die Streustrahlen signifikant und verbessert somit die Bildqualität, was wiederum grundsätzlich die Möglichkeit für eine geringere Dosisbelastung des Patienten schafft.

Spezifische Eigenschaften des Streustrahlenrasters, insbesondere basierend darauf, dass zwar eine komplexe Geometrie gegeben sein kann, aber sich im Ortsraum wiederholende geometrische Strukturen/Ausgestaltungen auftreten, bilden sich bei einer Transformation in den Frequenzraum mit geringer Unschärfe ab. Das Streustrahlenraster kennzeichnende Strukturen im Frequenzraum können so auf einfache Art und Weise identifiziert werden und das Kalibrierungsbild kann bezüglich der durch das Streustrahlenraster hervorgerufenen Bildmerkmale so angepasst werden, dass es möglichst optimal deckungsgleich über dem Röntgenbild liegt, so dass durch Subtraktion die Bildeffekte, insbesondere Artefakte, die durch das Streustrahlenraster entstehen, entfernt werden können. Die entsprechenden Anpassungen am Kalibrierungsbild werden hierbei durch Anpassungsparameter beschrieben, die auch im Frequenzraum möglicherweise aufgetretene Effekte beschreiben können, beispielsweise eine Verschiebung beziehungsweise Rotation einer Komponente der Aufnahmeanordnung und dergleichen.

Die Anpassungsparameter können dabei als freie Parameter in einem iterativen (Optimierungs-)Verfahren adaptiert werden, so dass adäquate Korrektur des aktuellen Röntgenbilds erfolgen kann, indem bevorzugt im Frequenzraum selber die entsprechenden Beiträge subtrahiert werden. Wie bereits erwähnt, handelt es sich bei den Anpassungsparametern um solche freie Parameter, die die Abweichung der unterschiedlichen Bild-Akquisitions-Zustände beschreiben, mithin die veränderte Situation zwischen der aktuellen Aufnahmegeometrie und der Aufnahmegeometrie bei der Kalibrierung der Röntgeneinrichtung, also der Aufnahme des Kalibrierungsbildes. Nach der Subtraktion des adaptierten Kalibrierungsbildes von dem aktuellen Röntgenbild im Frequenzraum wird das korrigierte Röntgenbild unter Nutzung der inversen Transformation wieder in den Ortsraum transformiert und kann dann entsprechend weiterverarbeitet werden.

Es sei angemerkt, dass selbstverständlich auch mit mehreren Kalibrierungsbildern gearbeitet werden kann. Das hier beschriebene Verfahren lässt sich ohne Einschränkungen der allgemeinen Gültigkeit bei allen bildgebenden Röntgeneinrichtungen anwenden, beispielsweise bei Röntgeneinrichtungen mit einem C-Bogen, CT-Einrichtungen, Radiographiesystemen und dergleichen.

Nachdem die Korrektur im Frequenzraum ermittelt wird, werden problematische Interpretationen im Ortsraum vermieden und die Bindung an die zuvor vorliegende Pixelmatrix des Röntgenbildes wird aufgehoben. Zudem entsprechen, wie noch genauer dargestellt werden soll, viele freie Parameter im Frequenzraum entsprechend auftretenden Veränderungen der Aufnahmegeometrie und/oder anderer Aufnahmeparameter, wie im Folgenden noch näher dargestellt werden wird.

So kann vorgesehen sein, dass als Anpassungsparameter die Phase des Kalibrierungsbildes im Ortsfrequenzraum und/oder die Intensität des Kalibrierungsbildes im Ortsfrequenzraum und/oder eine Rotation des Kalibrierungsbildes im Ortsfrequenzraum verwendet werden. Gerade bei der Anwendung in einer Röntgeneinrichtung mit einem C-Bogen unterliegen das Streustrahlenrasters sowie andere Anteile der Aufnahmeanordnung starken mechanischen Einflüssen, beispielsweise durch die unterschiedlichen Angulationen des C-Bogens. Dies kann leicht mechanische Verschiebungen beispielsweise des Streustrahlenrasters hervorrufen, die sich im Frequenzraum durch eine Phasenverschiebung darstellen, das bedeutet, die Artefaktbeiträge weisen eine Phasenverschiebung gegenüber dem eigentlichen Kalibrierungsbild auf. Wird mithin als Anpassungsparameter die Phase im Kalibrierungsbild im Ortsfrequenzraum betrachtet, können derartige Effekte leicht durch entsprechende Anpassung des Kalibrierungsbildes berücksichtigt werden. Auch die Intensität der Kalibrierungsbilder kann anzupassen sein, wenn beispielsweise das unterschiedliche Absorptionsverhalten von Patienten beziehungsweise allgemeinen Bildgebungsobjekten zu sehr unterschiedlichen Signalstärken am Röntgendetektor (inklusive Streustrahlenraster) führt. Aufgrund dieser unterschiedlichen Signalstärken stellen sich dann auch die Artefaktbeiträge mit unterschiedlicher Intensität dar, so dass zweckmäßigerweise ein Maß für die Intensität im Kalibrierungsbild im Frequenzraum als Anpassungsparameter herangezogen wird. Schließlich kann auch eine Rotation des Kalibrierungsbildes im Ortsfrequenzraum durch die Anpassungsparameter beschrieben werden, nachdem sich eine Rotation im Ortsraum auch als eine Rotation im Ortsfrequenzraum darstellen lässt. Selbstverständlich sind auch weitere Anpassungsparameter grundsätzlich denkbar.

Als Optimierungsverfahren zur Bestimmung der optimalen Werte für die Anpassungsparameter und mithin des optimal angepassten Kalibrierungsbildes können grundsätzlich bekannte Optimierungsverfahren eingesetzt werden, beispielsweise gradientenbasierte Optimierungsverfahren und/oder Newton-Optimierungsverfahren.

Als Transformation kann eine übliche, zweidimensionale Fouriertransformation verwendet werden. Bevorzugt ist es im Rahmen der vorliegenden Erfindung jedoch, wenn eine wenigstens eine Geometrieeigenschaft des Streustrahlenrasters berücksichtigende Geometrietransformation, insbesondere eine Wavelet-Transformation, als die Transformation verwendet wird. Dabei kann zweckmäßigerweise ein dediziertes Wavelet angewendet werden, welches eine entsprechende Korrespondenz mit der sich periodisch räumlich wiederholenden geometrischen Ausgestaltung, insbesondere einer in einem Grid-Muster wiederholten geometrischen Struktur, aufweist. Dies ermöglicht es, die Unschärfe, mit der das Kalibrierungsbild im Frequenzraum abgebildet wird, zu minimieren, so dass gewährleistet werden kann, dass sich die Bildkorrektur auf die charakteristischen Frequenzen beschränkt, die durch das Streustrahlenraster und konkret dessen sich periodisch räumlich wiederholende geometrische Ausgestaltung induziert wurden. Konkret kann hierbei vorgesehen sein, dass als Wavelet einer Wavelet-Transformation eine ein sich periodisch wiederholendes geometrisches Grundmuster des Streustrahlenrasters beschreibende Funktion verwendet wird. Im Rahmen der Wavelet-Transformation oder allgemein der Geometrietransformation kann eine Korrelationsrechnung mit einem Signal, im konkreten Beispiel dem Wavelet, das der geometrischen Struktur, beispielsweise einer Wabe bei einem Wabenmuster des Streustrahlenrasters, entspricht, durchgeführt werden. Durch diese Korrelation, die bevorzugt, wie grundsätzlich bekannt, in mehreren Skalierungsschritten durchgeführt wird, ist letztlich schon eine Art von Optimierung gegeben, so dass zweckmäßigerweise vorgesehen sein kann, dass die Ermittlung der Anpassungsparameter wenigstens teilweise im Rahmen der Durchführung der Wavelet-Transformation erfolgt.

Ein weiterer Vorteil der Verwendung einer Geometrietransformation, oder speziell einer Wavelet-Transformation, ist, dass diese bereits per se lokal ausgestaltet werden kann. Allerdings ist es im Rahmen der vorliegenden Erfindung auch grundsätzlich zweckmäßig, wenn die Transformation lokal angewendet wird, insbesondere als gefensterte Transformation. Auf diese Weise können beispielsweise Rauscheffekte, insbesondere in Randbereichen des Röntgenbildes, besser behandelt werden, so dass letztlich eine lokale Optimierung möglich ist. Beispielsweise kann also eine gefensterte zweidimensionale Fourier Transformation eingesetzt werden.

Hierbei ist es zweckmäßig, wenn eine zur lokalen Anwendung verwendete Fensterfunktion eine Ausdehnung eines geometrischen Grundmusters des Streustrahlenrasters oder ein Vielfaches dieses Wertes aufweist und/oder als die Form des Grundmusters oder eine dieses umschreibende Zelle abbildend gewählt wird. Das Grundmuster entspricht dabei, wie bereits erwähnt, der sich räumlich periodisch wiederholenden Struktur beziehungsweise Ausgestaltung, beispielsweise einer Wabe bei Verwendung beispielsweise eines aus Waben gebildeten Gitters (Grid-Struktur). Mithin weist zweckmäßiger Weise eine entsprechende Fenstertransformation eine adäquate Korrespondenz mit einem Grid-Muster auf, so dass beispielsweise die Form der Fensterfunktion identisch dem periodisch räumlich wiederholten Grid-Muster sein kann; dabei kann vorgesehen sein, dass die tatsächliche Größe der Fensterfunktion, also deren Ausdehnung, mit einem entsprechenden Vergrößerungsfaktor behaftet wird.

Eine besonders vorteilhafte Weiterbildung der vorliegenden Erfindung sieht vor, dass als Startwerte für die zu optimierenden Anpassungsparameter Ergebnisse einer vorherigen Korrektur an einem Vorabbild verwendet werden. Mithin kann das erfindungsgemäße Verfahren durch eine Art Speicherelement erweitert werden, was es ermöglicht, das adaptive Vorgehen, also die Bestimmung der das Übereinstimmungsmaß maximierenden Anpassungsparameter, adäquat zu initialisieren. Dabei sind verschiedene Ausgestaltungen denkbar.

So kann zum einen vorgesehen sein, dass als Vorabbild ein unmittelbar vor dem aktuellen Röntgenbild aufgenommenes Röntgenbild verwendet wird. Mithin werden die als optimierend bestimmten Ergebniswerte der Anpassungsparameter einer unmittelbar vorangehenden Aufnahme, beispielsweise bei der Fluoroskopie und/oder wiederholten Aufnahmen, als Startwerte für die Anpassung des Kalibrierungsbildes auf das aktuelle (Live-)Röntgenbild verwendet, so dass mithin davon ausgegangen wird, dass nur eine kleine Veränderung zu besorgen ist.

Besonders bevorzugt ist es im Rahmen der Erfindung jedoch, wenn insbesondere in einem Kalibrierungsvorgang für verschiedene insbesondere die Aufnahmegeometrie beschreibende Aufnahmeparameter der Röntgeneinrichtung Vorabbilder aufgenommen und optimierte Anpassungsparameter bestimmt und den Aufnahmeparametern zugeordnet als Datenbank abgespeichert werden, wobei die Startwerte für das aktuelle Röntgenbild entsprechend den aktuellen Aufnahmeparametern aus der Datenbank gewählt werden. In dieser Ausgestaltung wird also ein weiterer Kalibrierungsvorgang vorgesehen, um ein Speicherelement mit einer Datenbank zu befüllen, in der wenigstens teilweise die Aufnahmegeometrie beschreibenden Aufnahmeparametern Startwerte zur Anpassung des Kalibrierungsbilds auf das aktuelle Röntgenbild im Ortsfrequenzraum zugeordnet sind. Liegen die Aufnahmeparameter in der Datenbank identisch vor, können unmittelbar die Startwerte verwendet werden; es ist jedoch auch denkbar, immer die den nächstliegend an den aktuellen Aufnahmeparametern bestimmten Aufnahmeparametern der Datenbank zugeordneten Startwerte zu verwenden und/oder eine Interpolation beziehungsweise Extrapolation durchzuführen.

Die Datenbank bildet mithin eine Art Look-Up-Tabelle. Auf diese Weise könne mit besonderem Vorteil charakteristische Abhängigkeiten von den Aufnahmeparametern berücksichtigt werden und als Startpunkt der Adaption übergeben werden. Dabei kann auch über die Aufnahmegeometrie hinaus eine Betrachtung von Aufnahmeparametern erfolgen, beispielsweise eine Abhängigkeit der Intensität vom aktuellen Absorptionsverhalten des durchleuchteten Bildgebungsobjekts betrachtet werden. Auf diese Weise ist eine verlässliche, robuste und schnellere Ermittlung der optimierenden Werte der Anpassungsparameter möglich.

Neben dem Verfahren betrifft die Erfindung auch eine Röntgeneinrichtung mit den Merkmalen des Anspruchs 10, aufweisend eine zur Durchführung des erfindungsgemäßen Verfahrens ausgebildete Steuereinrichtung. So kann die Korrektur von Effekten eines Streustrahlenrasters der Röntgeneinrichtung, welches als Teil einer Aufnahmeanordnung mit einem Röntgenstrahler und einem Röntgendetektor detektorseitig vorgesehen sein kann, gleich an der Röntgeneinrichtung korrigiert werden. Bei der Röntgeneinrichtung kann es sich insbesondere um eine Röntgeneinrichtung mit einem C-Bogen handeln, an dem sich gegenüberliegend ein Röntgenstrahler und ein Röntgendetektor angeordnet sind. Das Streustrahlenraster kann beispielsweise dem Röntgendetektor vorgeschaltet auf diesen aufgesetzt beziehungsweise an diesem gehaltert sein. Sämtliche Ausführungen bezüglich des erfindungsgemäßen Verfahrens lassen sich analog auf die erfindungsgemäße Röntgeneinrichtung übertragen, mit welcher mithin ebenso die bereits genannten Vorteile erhalten werden können.

Ein erfindungsgemäßes Computerprogramm ist beispielsweise direkt in einen Speicher einer Steuereinrichtung einer Röntgeneinrichtung ladbar und weist Programmmittel auf, um die Schritte eines hierin beschriebenen Verfahrens auszuführen, wenn das Computerprogramm in der Steuereinrichtung der Röntgeneinrichtung ausgeführt wird. Werden dem Computerprogramm das Kalibrierungsbild und das Röntgenbild bereitgestellt, kann es selbstverständlich auch auf einer anderen Recheneinrichtung ausgeführt werden, beispielsweise an einem Auswertungsarbeitsplatz. Das Computerprogramm kann auf einem erfindungsgemäßen elektronisch lesbaren Datenträger gespeichert sein, der darauf gespeicherte elektronisch lesbare Steuerinformationen umfasst, welche zumindest das genannte Computerprogramm umfassen und derart ausgestaltet sind, dass sie bei Verwendung des Datenträgers in einer Steuereinrichtung einer Röntgeneinrichtung oder einer sonstigen Recheneinrichtung ein hierin beschriebenes Verfahren durchführen. Auch bezüglich des Computerprogramms und des Datenträgers gelten die Ausführungen zum erfindungsgemäßen Verfahren fort.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines ersten Ausführungsbeispiels des erfindungsgemäßen Verfahrens,
- Fig. 2: einen Ablaufplan eines zweiten Ausführungsbeispiels des erfindungsgemäßen Verfahrens, und
- Fig. 3: eine erfindungsgemäße Röntgeneinrichtung.

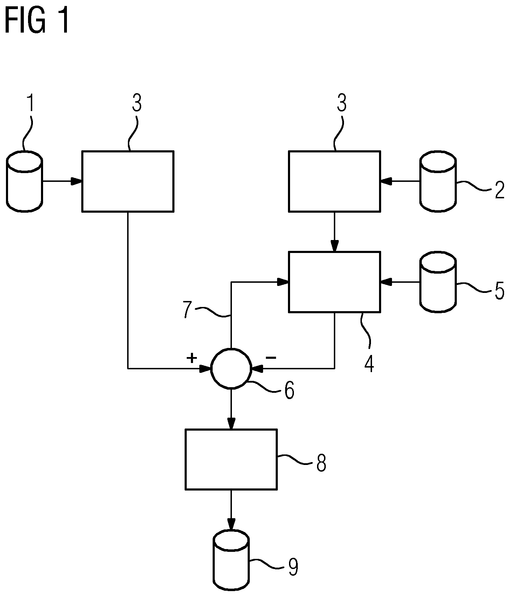

Fig. 1 zeigt einen Ablaufplan eines ersten Ausführungsbeispiels des erfindungsgemäßen Verfahrens. Ausgangspunkt ist hierbei ein Röntgenbild 1, das mit einer Röntgeneinrichtung unter Verwendung eines Streustrahlenrasters aufgenommen wurde und das mit Hilfe eines Kalibrierungsbildes 2, das nur Effekte des Streustrahlenrasters zeigt, also ohne Bildgebungsobjekt aufgenommen wurde, korrigiert werden soll. Nachdem Veränderungen in der Aufnahmesituation zwischen den Aufnahmen des Kalibrierungsbildes 2, das in einer Speichereinrichtung der Steuereinrichtung der Röntgeneinrichtung gespeichert sein kann, und dem Röntgenbild 1 auftreten können, muss das Kalibrierungsbild 2 erst adaptiert werden, bevor es zur Korrektur durch Subtraktion vom Röntgenbild 1 verwendet werden kann. Hierzu werden sowohl das Röntgenbild 1 als auch das Kalibrierungsbild 2 gemäß der Schritte 3 zunächst in den ortsfrequenzraum transformiert, wobei als Transformation hier eine gefensterte zweidimensionale Fouriertransformation verwendet wird. Das hier verwendete Streustrahlenraster weist sich periodisch nach Art eines Gitters ("Grid") wiederholende Grundmuster, beispielsweise Waben, auf. Die Fensterfunktion orientiert sich in ihrer Ausdehnung und Signalform an diesen Waben, um eine lokale Optimierung zu ermöglichen.

Wurden das Röntgenbild 1 und das Kalibrierungsbild 2 erst in den Ortsfrequenzraum transformiert, wird das Kalibrierungsbild 2 gemäß dem Schritt 4 adaptiert. Das bedeutet, es werden ein Übereinstimmungsmaß zwischen dem Röntgenbild 1 und dem Kalibrierungsbild 2 optimierende Anpassungsparameter, die die Adaption des Kalibrierungsbilds 2 beschreiben, in einem Optimierungsverfahren ermittelt.

Dazu werden nun zunächst Startwerte aus einer Datenbank 5 gewählt. Die Datenbank 5 enthält geeignete Startwerte, die Sätzen von Aufnahmeparametern zugeordnet sind. Die dem den aktuellen Aufnahmeparametern am besten entsprechenden Aufnahmeparametersatz zugeordneten Startwerte werden zu Beginn der Adaptierung im Schritt 4 ausgewählt und angewendet. Wie durch den Kreis 6 angedeutet, wird sodann das mittels der Startwerte angepasste Kalibrierungsbild 2 von dem Röntgenbild 1 abgezogen, und zwar im Ortsfrequenzraum. Die Maximierung des Übereinstimmungsgrades entspricht hier also einer Minimierung der Differenz. Die Optimierung, welche nach einem üblichen Optimierungsverfahren erfolgen kann, wird dabei durch den Pfeil 7 symbolisiert.

Als Anpassungsparameter werden vorliegend die Phase des Kalibrierungsbildes 2, die Intensität des Kalibrierungsbildes 2 und eine Rotation des Kalibrierungsbildes 2, jeweils im Frequenzraum, betrachtet. Eine Phasenverschiebung entspricht einer Verschiebung im Ortsraum, Intensitätsänderungen können sich durch das zu durchstrahlende Bildgebungsobjekt, insbesondere einen Patienten, ergeben und eine Rotation im Frequenzraum bildet eine Rotation im Ortsraum ab.

Wird durch ein Abbruchkriterium des Optimierungsverfahrens eine hinreichende Maximierung des Übereinstimmungsmaßes festgestellt, ist die Adaption des Kalibrierungsbildes 2 abgeschlossen und die entsprechende Differenz bildet das korrigierte Röntgenbild im Ortsfrequenzraum. Mithin wird nun das Inverse der gefensterten zweidimensionalen Fouriertransformation des Schrittes 3 in einem Schritt 8 angewandt, um das korrigierte Röntgenbild 9 im Ortsraum zu erhalten.

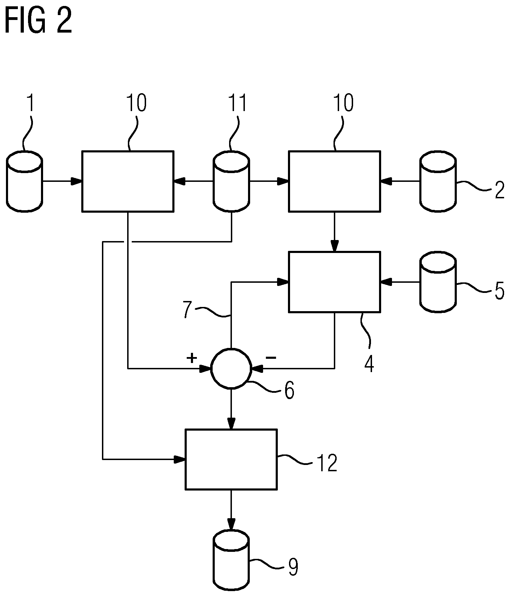

Fig. 2 zeigt eine weitere Ausführungsform des erfindungsgemäßen Verfahrens, die sich von der Ausführungsform der Fig. 1 in der Art der Transformation in den Ortsfrequenzraum unterscheidet. Hier wird im Schritt 10 eine Wavelet-Transformation angewendet, wobei das verwendete Wavelet 11 unter Nutzung von Hintergrundwissen über das Streustrahlenraster ausgewählt wurde. Beispielsweise kann die Signalform des Wavelet dem bereits erwähnten, räumlich periodisch wiederholten Grundmuster im Streustrahlenraster entsprechen. Auf diese Weise ist es möglich, die durch das Streustrahlenraster 11 induzierten Frequenzstrukturen besonders deutlich im Ortsfrequenzraum zu erhalten, mithin im Wesentlichen auf die durch das Streustrahlenraster induzierten Bildeffekte, insbesondere Artefakte, zu fokussieren und somit eine verbesserte Optimierung zu erreichen, die teilweise auch bereits im Rahmen der Transformation selbst stattfinden kann, was der Übersichtlichkeit halber hier nicht näher dargestellt ist. Entsprechend wird im Schritt 12 auch die inverse Wavelet-Transformation angewendet, um das korrigierte Röntgenbild 9 zu erhalten.

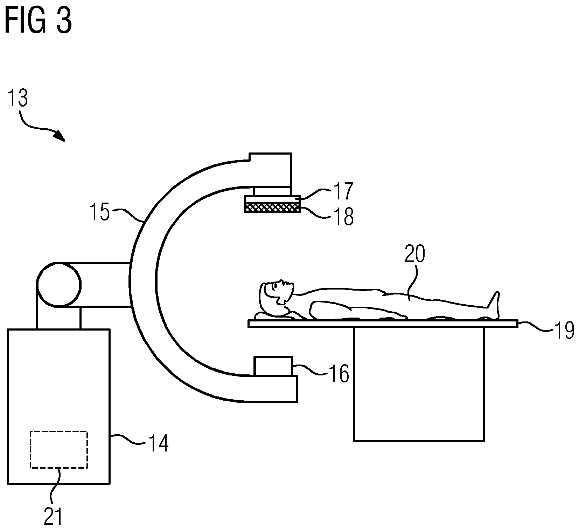

Fig. 3 zeigt schließlich eine Prinzipskizze einer erfindungsgemäßen Röntgeneinrichtung 13. Diese umfasst einen an einem Stativ 14 angeordneten C-Bogen 15, an dem sich gegenüberliegenden ein Röntgenstrahler 16 und ein Röntgendetektor 17 angeordnet sind. An dem Röntgendetektor 17 gehaltert ist vorliegend ein Streustrahlenraster 18 angeordnet, das eine sich räumlich periodisch wiederholende Struktur, also ein geometrisches Grundmuster, nach Art eines Gitters aufweist.

Mittels des C-Bogens 15 kann die durch den Röntgenstrahler 16 und den Röntgendetektor 17 gebildete Aufnahmeanordnung in verschiedene, Aufnahmegeometrien definierende Aufnahmepositionen bezüglich eines auf einem Patiententisch 19 gelagerten Patienten 20 verfahren werden.

Der Betrieb der Röntgeneinrichtung 13 wird über eine Steuereinrichtung 21 gesteuert, die zur Durchführung des erfindungsgemäßen Verfahrens ausgebildet ist. Das bedeutet, immer dann, wenn ein neues Röntgenbild aufgenommen wurde, wird ein in einer Speichereinrichtung der Steuereinrichtung 21 gespeichertes Kalibrierungsbild im Frequenzraum so adaptiert, dass eine bestmögliche Korrektur der von dem Streustrahlenraster 18 hervorgerufenen Bildeffekte, insbesondere Artefakte, erfolgt.

Ein hierin beschriebenes Verfahren kann auch in Form eines Computerprogramms vorliegen, dass das Verfahren auf der Steuereinrichtung 21 implementiert, wenn es auf der Steuereinrichtung 21 ausgeführt wird. Ebenso kann ein elektronisch lesbarer Datenträger (nicht dargestellt) mit darauf gespeicherten elektronisch lesbaren Steuerinformationen vorliegen, welche zumindest ein beschriebenes Computerprogramm umfassen und derart ausgestaltet sind, dass sie bei Verwendung des Datenträgers in der Steuereinrichtung 21 der Röntgeneinrichtung 13 ein beschriebenes Verfahren durchführen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Korrektur eines mit einer Röntgeneinrichtung (13) mit einem Streustrahlenraster (18) aufgenommenen Röntgenbilds (1) auf Effekte des Streustrahlenrasters (18), wobei das Streustrahlenraster (18) eine sich räumlich periodisch wiederholende geometrische Ausgestaltung aufweist und ein ohne Bildgebungsobjekt aufgenommenes Kalibrierungsbild (2) verwendet wird, **dadurch gekennzeichnet, dass** das Kalibrierungsbild (2) und das Röntgenbild (1) mittels einer Transformation in den Ortsfrequenzraum transformiert werden, im Ortsfrequenzraum ein Übereinstimmungsmaß zwischen dem Röntgenbild (1) und dem Kalibrierungsbild (2) optimierende Veränderungen des Kalibrierungsbildes (2) beschreibende Anpassungsparameter ermittelt werden, zur Korrektur das angepasste Kalibrierungsbild (2) von dem Röntgenbild subtrahiert (1) wird und das aus dieser Subtraktion erhaltene, korrigierte Röntgenbild (1) durch Anwendung des Inversen der Transformation wieder in den Ortsraum zurücktransformiert wird.

2. Verfahren nach Anspruch 1, **dadurch gekennzeichnet, dass** als Anpassungsparameter die Phase des Kalibrierungsbildes (2) im Ortfrequenzraum und/oder die Intensität des Kalibrierungsbildes (2) im Ortsfrequenzraum und/oder eine Rotation des Kalibrierungsbildes (2) im Ortsfrequenzraum verwendet werden.

3. Verfahren nach Anspruch 1 oder 2, **dadurch gekennzeichnet, dass** als Transformation eine zweidimensionale Fouriertransformation oder eine wenigstens eine Geometrieeigenschaft des Streustrahlenrasters (18) berücksichtigende Geometrietransformation verwendet wird.

4. Verfahren nach Anspruch 3, **dadurch gekennzeichnet, dass** als Wavelet einer Wavelet-Transformation eine ein sich periodisch wiederholendes geometrisches Grundmuster des Streustrahlenrasters (18) beschreibende Funktion verwendet wird und/oder die Ermittlung der Anpassungsparameter wenigstens teilweise im Rahmen der Durchführung der Wavelet-Transformation erfolgt.

5. Verfahren nach einem der vorangehenden Ansprüche, **dadurch gekennzeichnet, dass** die Transformation lokal angewendet wird.

6. Verfahren nach Anspruch 5, **dadurch gekennzeichnet, dass** eine zur lokalen Anwendung verwendete Fensterfunktion eine Ausdehnung eines geometrischen Grundmusters des Streustrahlenrasters (18) oder ein Vielfaches dieses Wertes aufweist und/oder als die Form des Grundmusters oder eine dieses umschreibende Zelle abbildend gewählt wird.

7. Verfahren nach einem der vorangehenden Ansprüche, **dadurch gekennzeichnet, dass** als Startwerte für die zu optimierenden Anpassungsparameter Ergebnisse einer vorherigen Korrektur an einem Vorabbild verwendet werden.

8. Verfahren nach Anspruch 7, **dadurch gekennzeichnet, dass** als Vorabbild ein unmittelbar vor dem aktuellen Röntgenbild (1) aufgenommenes Röntgenbild verwendet wird.

9. Verfahren nach Anspruch 7, **dadurch gekennzeichnet, dass** in einem Kalibrierungsvorgang für verschiedene die Aufnahmegeometrie beschreibende Aufnahmeparameter der Röntgeneinrichtung (13) Vorabbilder aufgenommen und optimierte Anpassungsparameter bestimmt und den Aufnahmeparametern zugeordnet als Datenbank (5) abgespeichert werden, wobei die Startwerte für das aktuelle Röntgenbild (1) entsprechend den aktuellen Aufnahmeparametern aus der Datenbank (5) gewählt werden.

10. Röntgeneinrichtung (13), aufweisend eine Aufnahmeanordnung mit einem Röntgenstrahler (16), einem Röntgendetektor (17) und einem detektorseitig vorgesehenen Streustrahlenraster (18) und eine zur Durchführung eines Verfahrens nach einem der vorangehenden Ansprüche ausgebildete Steuereinrichtung (21) mit einer Speichereinrichtung.

11. Computerprogramm, welches die Schritte eines Verfahrens nach einem der Ansprüche 1 bis 9 durchführt, wenn es auf einer Recheneinrichtung ausgeführt wird.

12. Elektronisch lesbarer Datenträger, auf dem ein Computerprogramm nach Anspruch 11 gespeichert ist.

## Claims

1. Method for correction of an x-ray image (1) recorded with an x-ray device (13) with an anti-scatter grid (18) for effects of the anti-scatter grid (18), wherein the anti-scatter grid (18) has a spatially periodically repeating geometrical embodiment and a calibration image (2) recorded without an imaging object is used, **characterised in that** the calibration image (2) and the x-ray image (1) will be transformed by means of a transformation in the position frequency space, in the position frequency space adaptation parameters describing changes to the calibration image (2) optimizing a measure of matching between the x-ray image (1) and the calibration image (2) will be established, for correction the adapted calibration image (2) will be subtracted from the x-ray image (1) and the corrected x-ray image (1) obtained from this subtraction will be transformed back into the position space again by using the inverse of the transformation.

2. Method according to claim 1, **characterised in that** the phase of the calibration image (2) in the position frequency space and/or the intensity of the calibration image (2) in the position frequency space and/or a rotation of the calibration image (2) in the position frequency space will be used as adaptation parameters.

3. Method according to claim 1 or 2, **characterised in that** a two-dimensional Fourier transform or a geometry transformation taking account of at least one geometry characteristic of the anti-scatter grid (18) will be used as the transformation.

4. Method according to claim 3, **characterised in that** a function describing a periodically repeating geometric pattern of the anti-scatter grid (18) will be used as the wavelet of a wavelet transformation and/or the adaptation parameters are established at least partly within the framework of carrying out the wavelet transformation.

5. Method according to one of the preceding claims, **characterised in that** the transformation will be applied locally.

6. Method according to claim 5, **characterised in that** a window function used for local application has an extension of a geometrical basic pattern of the anti-scatter grid (18) or a multiple of this value and/or will be selected as mapping the shape of the basic pattern or of a cell circumscribing said pattern.

7. Method according to one of the preceding claims, **characterised in that** results of a previous correction to a preliminary image will be used as start values for the adaptation parameters to be optimized.

8. Method according to claim 7, **characterised in that** an x-ray image recorded immediately before the current x-ray image (1) will be used as a preliminary image.

9. Method according to claim 7, **characterised in that**, in particular in a calibration process for different recording parameters of the x-ray device (13) describing the recording geometry, preliminary images will be recorded and optimized adaptation parameters will be determined and stored assigned to the recording parameters as a database (5), wherein the start values for the current x-ray image (1) will be selected from the database (5) in accordance with the current recording parameters.

10. X-ray device (13), having a recording arrangement with an x-ray emitter (16), an x-ray detector (17) and an anti-scatter grid (18) provided on the detector side and a control device (21), with a memory device, embodied for carrying out a method according to one of the preceding claims.

11. Computer program, which carries out the steps of a method according to one of claims 1 to 9, when it is executed on a computing device.

12. Electronically-readable data medium, on which a computer program according to claim 11 is stored.

## Revendications

1. Procédé de correction d'une radiographie (1), enregistrée par un dispositif (13) radiographique ayant une grille (18) d'anti-diffusion, des effets de la grille (18) d'anti-diffusion, dans lequel la grille (18) d'anti-diffusion a une conformation géométrique se répétant périodiquement dans l'espace et on utilise une image (2) d'étalonnage enregistrée sans objet d'imagerie, **caractérisé en ce que** l'on transforme l'image (2) d'étalonnage et la radiographie (1) au moyen d'une transformation dans l'espace lieu-fréquence, on détermine, dans l'espace lieu-fréquence, des paramètres d'adaptation décrivant des modifications de l'image (2) d'étalonnage optimisant un degré de coïncidence entre la radiographie (1) et l'image d'étalonnage, pour la correction, on soustrait (1) l'image (2) d'étalonnage adaptée de la radiographie et on retransforme à nouveau dans l'espace lieu, en utilisant l'inverse de la transformation, l'image (1) radiographique corrigée obtenue à partir de cette soustraction.

2. Procédé suivant la revendication 1, **caractérisé en ce que** l'on utilise, comme paramètre d'adaptation, la phase de l'image (2) d'étalonnage dans l'espace lieu-fréquence et/ou l'intensité de l'image (2) d'étalonnage dans l'espace lieu-fréquence et/ou une rotation de l'image (2) d'étalonnage dans l'espace lieu-fréquence.

3. Procédé suivant la revendication 1 ou 2, **caractérisé en ce que** l'on utilise, comme transformation, une transformation de Fourier à deux dimensions ou une transformation géométrique prenant en compte au moins une propriété géométrique de la grille (18) d'anti-diffusion.

4. Procédé suivant la revendication 3, **caractérisé en ce que** l'on utilise, comme ondelettes d'une transformation d'ondelette, une fonction décrivant un motif géométrique de base de la grille (18) d'anti-diffusion se reproduisant périodiquement et/ou on effectue la détermination des paramètres d'adaptation, au moins en partie dans le cadre de la mise en oeuvre de la transformation d'ondelette.

5. Procédé suivant l'une des revendications précédentes, **caractérisé en ce que** l'on applique la transformation localement.

6. Procédé suivant la revendication 5, **caractérisé en ce qu'**une fonction fenêtre utilisée pour l'application locale a une étendue d'un motif géométrique de base de la grille (18) d'anti-diffusion ou un multiple de cette valeur et/ou on la choisit de manière à reproduire la forme du motif de base ou une cellule le circonscrivant.

7. Procédé suivant l'une des revendications précédentes, **caractérisé en ce que** l'on utilise, comme valeurs de départ des paramètres d'adaptation à optimiser, des résultats d'une correction précédente sur une image préalable.

8. Procédé suivant la revendication 7, **caractérisé en ce que** l'on utilise, comme image précédente, une radiographie enregistrée juste avant la radiographie en cours.

9. Procédé suivant la revendication 7, **caractérisé en ce que**, dans une opération d'étalonnage de divers paramètres d'enregistrement décrivant la géométrie d'enregistrement du dispositif (13) radiographique, on enregistre des images préalables et on détermine des paramètres d'adaptation optimisés et associés aux paramètres d'enregistrement, on les mémorise sous forme de base (5) de données, les valeurs de départ pour la radiographie (1) en cours étant choisies dans la base (5) de données conformément aux paramètres d'enregistrement en cours.

10. Appareil (13) radiographique ayant un agencement d'enregistrement comprenant un émetteur (16) de rayons X, un détecteur (17) de rayons X et une grille (18) d'anti-diffusion prévue du côté du détecteur et un dispositif (21) de commande constitué pour effectuer un procédé suivant l'une des revendications précédentes et ayant un dispositif de mémoire.

11. Programme d'ordinateur qui effectue les stades d'un procédé suivant l'une des revendications 1 à 9 lorsqu'il est exécuté sur un dispositif informatique.

12. Support de données déchiffrables électroniquement, sur lequel un programme d'ordinateur suivant la revendication 11 est mémorisé.
